# EUROPEAN PATENT APPLICATION

(11) **EP 4 094 594 A1**
(43) Date of publication of application: **30.11.2022**
(21) Application number: 22466001.9
(22) Date of filing: 19.05.2022
(51) Int. Cl.: A24B 15/16, A24B 15/24, A61P 25/34, A24B 15/28, C07D 401/04, A61K 31/465, A61K 9/00, A24B 13/00

(54) **A FILING OF A NICOTINE FORMULATION, IN PARTICULAR A NICOTINE SACHET AND THE METHOD OF ITS MANUFACTURE, AND A MIXTURE OF NICOTINE SALTS AND THE METHOD OF ITS MANUFACTURE**

(30) Priority: 25.05.2021 CZ 202100255 U; 25.05.2021 CZ 202100256 U
(71) Applicant: Consumer Brands International s.r.o., 415 03 Retenice (CZ)
(72) Inventor: Pfleger, Richard, 120 00 Praha 2 (CZ)
(74) Representative: Plicka, Josef

(57) **Abstract**

The invention relates to a filling of a nicotine composition, in particular a sachet, the subject matter of which is that it comprises from 0.0001 to 10 parts by weight of a mixture of nicotine salts, comprising from 0.1 to 15 parts by weight of an electrolyte having a pH of from 2.4 to 12.2 and an oxidation-reduction potential of from minus 900 mV to plus 1200 mV, from 0.1 to 5 parts by weight of nicotine base, from 0, 01 to 3 parts by weight of excipients and 77 to 99, 79 parts by weight of water, 1 to 80 parts by weight of filler, 0.1 to 5 parts by weight of surfactants, 0.01 to 10 parts by weight of nicotine formulation fluid and colloidal enhancement agents, and 0.01 to 3 parts by weight of nicotine formulation absorption enhancement agents.

The invention further relates to a method of producing a nicotine formulation filling, the subject matter of which is to first mix the individual bulk fractions of the nicotine formulation, i.e. the filler, with the surfactants, agents to improve the fluid and colloidal properties and agents to improve the absorbability of the nicotine salt mixture by means of at least one vertical or inclined homogenizer, and then spraying the surface of the already homogenized powder fractions with the liquid nicotine salt mixture, comprising from 0,1 to 15 parts by weight of an electrolyte having a pH of from 2,4 to 12,2 and an oxidation-reduction potential of from minus 900 mV to plus 1200 mV, from 0,1 to 5 parts by weight of nicotine base, from 0,01 to 3 parts by weight of excipients, and 77 to 99, 79 parts by weight of water to produce a bulk nicotine formulation having a moisture content of from 1 to 50%.

The invention relates to a mixture of nicotine salts, the subject matter of which consists in that it comprises from 0.1 to 15 parts by weight of an electrolyte having a pH from 2.4 to 12.2 and an oxidation-reduction potential from minus 900 mV to plus 1200 mV, from 0.1 to 5 parts by weight of nicotine base, from 0.01 to 3 parts by weight of excipients and 77 to 99.79 parts by weight of water.

The invention further relates to a method of producing a mixture of nicotine salts, the principle of which is to mix an electrolyte having a pH of from 2.4 to 12.2 and an oxidation-reduction potential of from minus 900 mV to plus 1200 mV with water, nicotine base and excipients, whereby the water insoluble excipients are converted to a suspension and the mixture is then allowed to mature at a temperature of up to 20 degrees Celsius for a period of between six and forty-eight hours.

## Description

### Technical Field

The invention relates to a filling of a nicotine formulation with fillers, active surface binding agents, active surface binding agents, bulking and gliding agents, together with agents increasing the availability of nicotine through the nitrogen-containing mucosal barrier, and a method of producing it as a nicotine product for meeting the needs of persons desiring nicotine products.

The invention further relates to a mixture of nicotine salts and to a method of producing the mixture of nicotine salts, wherein the mixture of nicotine salts is produced as an intermediate product for the end production of, in particular, nicotine products such as nicotine sachets.

### Background Art

Cigarettes, cigars and a range of other alternative nicotine-containing products are currently used to meet the needs of persons desiring nicotine products. More recently, the development of nicotine products has focused on nicotine products that reduce the undesirable, particularly health-related, effects of cigarettes while providing users with a complete nicotine supply.

The starting materials for such nicotine products are generally mixtures of nicotine salts incorporating a nicotine base, but which is easily oxidised and unstable, and therefore efforts are made to bind the nicotine base to a non-covalent bond with other ingredients which eliminate the instability of the nicotine base.

The present solution for the filling of a nicotine composition, in particular nicotine sachets with fillers, and the method of producing the same, for example according to US 2015272878 A1, is marked by the multi-stage preparation of at least one free nicotine salt using a basic reagent, a solvent and the formation of a nicotine salt and its subsequent processing in the form of a granulate.

The resulting granulate is further utilized in a mixture with a filler, humectants, sensory correcting agents and pH adjusting agents to reconvert the stable salt to a free nicotine base in contact with saliva using a pH reducing agent.

In the case of granules, this is a multi-stage preparation involving the actual preparation of a stable form of nicotine salt from a nicotine base, which is unstable for its own use, and its technological processing and capture into fillers and packaging materials for buccal use is technologically difficult, particularly energy-intensive, or leads to insufficient and low absorption of nicotine across the mucosal membrane.

The subsequent preparation of the granulate is a technologically and time consuming step using physical methods, including dry or wet granulation, and these processes are characterised by high energy consumption, particularly in view of the need to achieve higher pressures. Existing production processes make heavy demands on the stability of the contents by drying, densification and subsequent costly processing.

The combination of granulate with filler and pH adjusting agents represents a further stage of production, involving cross-linking and homogenisation, with the risk of incomplete homogenisation.

The finalization of the physicochemical characteristics of the nicotine formulation filling by the addition of flavour correctors, sweeteners and humectants increases the risk of an unstable carrier environment, including the original physicochemical characteristics of the granulate or granulation is difficult in the state of the art, and often does not produce a sufficient degree of nicotine effect because the nicotine salts are not formed to a sufficient extent.

The prior art solutions either do not address or do not sufficiently address the issues associated with the oral mucosal barrier.

From an anatomical and histological point of view, the oral cavity is lined by a mucosa with a total surface area of approximately 100-200 cm². The buccal region of the oral cavity includes the inner cheeks and the inner regions of the upper and lower lip. This area occupies about one-third of the oral mucosa.

Under the tongue is the sublingual mucosa, which allows the rapid passage of substances into the blood, as the mucosa here is non-crusted, thin and perfectly blooded. On the hard palate and gums is the palatal mucosa containing cornified cells. These parts also contain neutral lipids - ceramides and acylceramides, which form an impermeable barrier. In contrast, the non-cartilaginous areas of the mucosa contain only small amounts of ceramides and allow more water to pass through than keratinized tissues. The physiological environment of the oral cavity is maintained by aqueous saliva, which is a suitable aqueous medium for the dissolution and subsequent absorption of the drug. Saliva regulates the oral microflora by maintaining a physiological pH in the mouth together with the activity of enzymes. The total production of saliva is 0.5 to 2.0 litres per day, depending on the physiology of the individual. Saliva has a pH of 5,5 to 7,0 and contains 99,5 % water. Mucus is produced in the goblet cells and salivary glands. The basic building blocks of mucus are the mucin glycoprotein. Together, these molecules are able to form three-dimensional structures carrying a negative charge and limiting transport across the epithelium.

The above characteristics of the oral cavity lead to the advantages of oral administration of nicotine products, in particular, good oral accessibility, suitable onset of nicotine base effects due to the high concentration gradient, so that a lower dose of nicotine base is required to meet the needs of nicotine effects.

Solutions according to the prior art use the oral cavity only as an application site for nicotine agents, without using the oral cavity to optimize the application of nicotine salts.

Document US 2005/0053665 A1 mentions nicotine that is bound to cellulose of non-seed origin. High concentrations of liquid free nicotine base are converted to chemically stable solid products via cellulose. The result, however, is a chemically unstable charge of nicotine products.

The starting materials for nicotine products of this nature are generally mixtures of nicotine salts incorporating a nicotine base, but the nicotine base is readily oxidised and unstable, and therefore efforts are made to bind the nicotine base to a non-covalent bond with other components which eliminate the instability of the nicotine base.

Existing solutions for the production and preparation of a nicotine salt mixture, for example, according to US 2015272878 A1 , are noted for the multistep preparation of at least one free nicotine salt using a basic reagent, a solvent, and the formation of a nicotine salt and its subsequent processing as a granulate.

The resulting granulate is further utilized in a mixture with a filler, humectants, sensory correcting agents and pH adjusting agents to reconvert the stable salt to a free nicotine base in contact with saliva using a pH reducing agent.

In the case of granules, this is a multi-stage preparation involving the actual preparation of a stable form of nicotine salt from a nicotine base which is, however, unstable for its own use and its technological processing and entrapment in fillers and packaging materials for buccal use is technologically difficult, particularly energy intensive, or leads to insufficient and low absorption of nicotine across the mucosal membrane.

The multi-stage preparation of nicotine salt described above is limited to only one form of nicotine salt. The preparation of a stable form of nicotine salt requires precise control of the pH by means of substances controlling the acidity or alkalinity of the mixture.

The subsequent preparation of the granulate is a technologically and time-consuming step using physical methods, including dry or wet granulation, and these processes are characterised by high energy consumption, particularly in view of the need to achieve higher pressures. Existing production processes make heavy demands on the stability of the contents by drying, densification and subsequent costly processing.

The combination of the granulate with the filler and with pH adjusting agents represents a further stage of production, involving cross-linking and homogenisation, with the risk of incomplete stabilisation of the nicotine salt and its homogenisation.

The finalization of the physicochemical characteristics by the addition of flavour correctors, sweeteners and humectants increases the risk of an unstable carrier environment, including the original physicochemical characteristics of the granulate or granulation.

Attempts to address these issues are evident, for example, in WO 2010/104 464 A1, which discloses an alginate salt matrix that traps a biologically active substance such as nicotine in the matrix. The nicotine in the form of nicotine salt is dissolved in the aqueous solution together with the alginate salt and other components. After drying of the solution, the nicotine is trapped with the other dissolved components in the alginate matrix, which provides protection for the nicotine and can be used to regulate the delivery of nicotine from the matrix.

The document US 2005/0053665 A1 mentions nicotine that is bound to cellulose of non-seed origin. High concentrations of liquid free nicotine base are converted to chemically stable solid products via cellulose.

### Summary of the Invention

The aforementioned shortcomings, difficulties, technical and manufacturing problems of the prior art can be eliminated by a solution according to the invention, the essence of which is that the nicotine formulation cartridge, which contains from 0.0001 to 10 parts by weight of a mixture of nicotine salts, which contains from 0.1 to 15 parts by weight of an electrolyte having a pH of from 2.4 to 12.2 and an oxidation-reduction potential of from minus 900 mV to plus 1200 mV, from 0, 1 to 5 parts by weight of nicotine base, from 0.01 to 3 parts by weight of excipients, and from 77 to 99, 79 parts by weight of water, from 1 to 80 parts by weight of filler, from 0.1 to 5 parts by weight of surfactants, from 0.01 to 10 parts by weight of nicotine formulation fluid and colloidal enhancement agents, and from 0.01 to 3 parts by weight of nicotine formulation absorption enhancement agents.

The filler is preferably a vegetable fibre, in particular cellulose, inulin, arabinogalactan, glucomannan, arabinoxylan or vegetable fructooligosaccharides, galactooligosaccharides or xylooligosaccharides.

The filler, or part thereof, may be in the form of microparticles ranging in size from 1 to 1000 µm.

Preferably, less than 8 weight percent of the filler has a particle size on the sieve of less than 60 micrometers and more than 45 weight percent of the filler has a particle size on the sieve of more than 400 micrometers.

The active surface substance is chitosan with the character of a natural ionex with a degree of deacetylation from 50% to 90%.

The substance for improving the fluidic and colloidal properties of the nicotine formulation is volcanic dust with a high proportion of diatomaceous earth, wherein the proportion of diatomaceous earth is 50 to 70 weight percent.

The substance for improving the absorbability of the nicotine mixture is a substance of terpenic character, in particular Korean pine needles and substances containing azane-type nitrogen.

The essence of the method of producing the nicotine formulation filler is that the individual powder fractions of the nicotine formulation, i.e. the fillers with the fluid and colloidal enhancement agents and the nicotine salt mixture absorption enhancement agents, are first mixed by means of at least one vertical or inclined homogenizer and then the surface of the already homogenized powder fractions is sprayed with the liquid nicotine salt mixture, comprising from 0,1 to 15 parts by weight of an electrolyte having a pH of from 2,4 to 12,2 and an oxidation-reduction potential of from minus 900 mV to plus 1200 mV, from 0,1 to 5 parts by weight of nicotine base, from 0,01 to 3 parts by weight of excipients, and 77 to 99, 79 parts by weight of water to produce a bulk nicotine formulation having a moisture content of from 1 to 50 %.

The various bulk fractions of the nicotine formulation are vigorously mixed by an auger and planetary mixer having a speed range of from 40 revolutions to 200 revolutions per minute, wherein the speed of the planetary motion arm is in the range of from 1 revolution to 10 revolutions per minute, and wherein mixing is carried out throughout the volume for at least 30 minutes by at least one inclined or vertical auger and planetary mixer.

The intermixed bulk fractions of the nicotine formulation are vigorously mixed for at least 30 minutes with the liquid mixture of nicotine salts.

The nicotine salt mixture according to the invention consists of from 0.1 to 15 parts by weight of an electrolyte having a pH of from 2.4 to 12.2 and an oxidation-reduction potential of from minus 900 mV to plus 1200 mV, from 0.1 to 5 parts by weight of nicotine base, from 0.01 to 3 parts by weight of excipients, and from 77 to 99, 79 parts by weight of water.

The excipients are at least one substance selected from the group consisting of monoterpenes, diterpenes, triterpenes, cyclic terpenes, carboxylic acid esters and cyclic ketones.

The subject matter of the method for producing the nicotine salt mixture according to the invention consists in mixing an electrolyte having a pH of from 2.4 to 12.2 and an oxidation-reduction potential of from minus 900 mV to plus 1200 mV with water, nicotine base and excipients, whereby the water insoluble excipients are converted to a suspension and the mixture is then allowed to mature at a temperature of up to 20 degrees Celsius for a period of between six and forty-eight hours.

The prepared mixture may be stirred vigorously for at least 15 minutes before maturing in its entire volume. The nicotine base is added to the electrolyte without light. The water-insoluble excipients are converted to a suspension by means of a high-speed mixer or cavitator for 3 to 8 minutes.

The prepared mixture is filtered after maturation to remove impurities and suspended solids with a grain size greater than 0,1 millimetre. The water is demineralised with a water softener containing strong acid catex resin before use in the nicotine salt mixture.

By combining the reaction of the aqueous electrolyte and the nicotine base, advantages are achieved based on the stabilization of nicotine to form a mixture of nicotine salts for the production of a nicotine delivery device by the buccal route, for example in the form of nicotine sachets. Nicotine salt mixtures prepared in this way can advantageously be used for the production of nicotine preparations in the form of sachets, as well as powders, tablets, dragees, capsules, lozenges, pellets or microparticles.

The formation and formation of the mixture of nicotine salts according to the invention represents a stable form of nicotine product. The nicotine salt mixtures thus obtained allow for optimal and sufficient absorption of nicotine. A reasonably rapid dissolution of the nicotine salt in the saliva of the user is achieved so that, with adjustment of the pH, all these forms are released gradually. Saliva is 99% water with a fluctuating pH of 7-8. The release thus occurs gradually according to the degree of ionization and concentration gradient of the salts.

The nicotine salt mixtures according to the invention lead to the advantages of oral administration, since the oral cavity is easily accessible and there is a rapid or massive onset of nicotine action due to the high concentration gradient, so that a lower dose is required for the same effect.

By combining the reaction of aqueous anolyte and catholyte with a nicotine base, the benefits are achieved based on the stabilisation of nicotine to form nicotine salts.

Other advantages of this solution are the ease of preparing an aqueous solution containing nicotine with the addition of all reactants including monoterpenes, diterpenes, triterpenes, cyclic terpenes, carboxylic acid esters, cyclic ketones, organic acids, polyalcohols, sweetener to form nicotine salts in situ and its subsequent incorporation into the filler mixture with low time and cost of forming the compositions.

The method of producing the mixture of nicotine salts according to the invention does not require energy and capital intensive equipment, in particular granulators and dryers. It contributes to improved occupational safety by minimizing the handling of the nicotine base, which is toxic.

The solution according to the present invention eliminates the difficulties of the prior art by solving the composition of the nicotine formulation and the method of its production. The nicotine blends according to the invention in combination with the filler composition and the method of producing them in combination with other excipients, i.e. a basic vegetable filler, substances increasing the active surface of the filler, i.e. in particular natural ionex, a technological treatment of the filler in the context of micropelletization which increases its binding capacity, substances modifying the fluidity and slipperiness of the mixture and substances increasing the absorption of nicotine from such a mixture with the filler, wherein at least one such substance contains an azanic form of nitrogen.

Even minute but demonstrable amounts of nicotine produce a significant pharmacodynamic effect on the user's central and peripheral nervous system.

The method of manufacture of nicotine sachets ensures optimum mixing of the liquid phase containing nicotine salts with fillers and additives to enable their smooth production, including nicotine absorption enhancers.

The prepared fill of nicotine agents meets the requirements of pH, flowability, slipperiness and moisture as key parameters necessary for the smooth adjustment of the bulk material containing nicotine salts into the sachets. The prepared nicotine formulation cartridge is used for the manufacture of final products for consumption by nicotine users in a variety of forms, for example, as a sachet, but also as powder, tablets, dragees, capsules, lozenges, pellets or microparticles.

The formation and formation of the cartridge of nicotine products according to the invention, represents a stable solid form thereof. The mixtures of fillers and other excipients thus obtained allow optimal and sufficient absorption of nicotine or its salts as an intermediate for the manufacture of sachets.

The mixtures of the various forms of fillers and excipients according to the invention lead to the advantages of oral administration, since the oral cavity is easily accessible and well blooded, and there is a rapid or massive onset of action of the nicotine due to the high concentration gradient, so that a lower dose of nicotine itself is required for the same effect. These facts require the use of substances that increase absorption across the mucosal barrier.

Other advantages of this solution are its ease of preparation of the filler in situ and its subsequent binding to the mixture with nicotine and its salts by the filler, with low time and financial costs for the formation of the compositions.

The method of producing the filler mixture for nicotine salts according to the invention does not require energy and capital intensive equipment, in particular granulators and dryers. Thus, it contributes to improving occupational safety by minimizing the handling of the nicotine base, which is toxic.

### Detailed Description of the Invention

The individual examples of the invention, which are given below, are based on the same substances and will therefore be described in more detail first in this section.

Thus, the exemplary embodiments of the invention, i.e., the nicotine cartridge, as a mixture of excipients and a mixture of nicotine salts, as well as the method of producing the nicotine cartridge, including the stability of these mixtures, predetermine the physicochemical properties of the critical components of these mixtures that are crucial to achieve the desired effect.

An essential component of the invention is a mixture of a filler with other substances having different physicochemical properties and substances promoting the absorption of nicotine in the mouth, to which, after perfect mixing of these components, which are in solid and bulk form, a mixture of nicotine salts in liquid form is subsequently added.

A form of plant fibre such as cellulose, inulin, arabinogalactan, glucomannan, arabinoxylan or other plant fructooligosaccharides, galactooligosaccharides or xylooligosaccharides may be used as a filler. The filler may be used in the form of micropelletized particles with a proportion of from 1 to 80 parts by weight of the nicotine formulation filling.

The substance for improving the fluidic and colloidal properties of the nicotine formulation cartridge is volcanic silica dust with high bulking, colloidal and fluidic properties at a proportion in the range of 0,01 to 10 parts by weight. The diatomaceous earth content reduces the surface tension of the powder mixture.

The active surface substance is chitosan with a content of 0.1 to 5 parts by weight, with the character of a natural ion exchange, with a degree of deacetylation ranging from 50% to 90%.

Chitosan and its derivatives are the best known absorption facilitators with a good safety profile. Chitosan is a positively charged linear polysaccharide produced by deacetylation from chitin present in crustacean shells and is available in a wide range of molecular weights, viscosity levels and degrees of deacetylation. Chitosan is insoluble at neutral and basic pH, but forms water-soluble salts with inorganic and organic acids. Chitosan is a bioadhesive and is able to react strongly with the negatively charged components of naso-/oropharyngeal epithelial cells and the overlying mucus layer, thus providing prolonged contact time for transport of substances across the membrane before the product is removed or degraded.

The ameliorative effect was greater when the chitosan had a higher molecular weight and a lower degree of deacetylation, i.e. a higher positive charge. Moreover, neither single nor long-term application of chitosan caused any apparent tissue damage to the mucosa.

Terpenic substances in the range of 0,01 to 3 parts by weight can be used as substances to increase absorption across the mucosal barrier.

Terpenic substances increase the permeability of membrane barriers. Typical representatives are - mono, - di, - tri terpenes and cyclic terpenes of plant nature such as d-limonene, geraniol, (+)- neomenthol, α pinene, 1,8 cineol, thymol, eucalyptol and others. Their action is based on the partial destruction of the lipid layer of the membrane barrier.

Korean pine needles (*Pinus koraiensis* Siebold) are a source of mono-saturated fatty acids, with a total saturated fatty acid content of 45,9 %, with a higher proportion of C12 lauric acid and C14 myristic acid, with a monounsaturated fatty acid content of 18 % and a polyunsaturated fatty acid content of 49,2 %. The proportion of volatile phenolic substances reaches 1% with a significant proportion of α-pinene (9.88%), β-caryophyllene (6.51%), D-limonene (2.5%) or dermacrene D (9.94%). These substances have a very high antioxidant capacity and act as lipid disruptors with anti-inflammatory and antimicrobial activity.

The mechanism of action of the absorption facilitators is based on the fact that it is provided by one or both of the following mechanisms.

The first is based on the fact that some compounds may alter the physicochemical properties of the drug substance in the formulation, e.g. by changing the solubility, partition coefficient or by weak ionic interactions with the active substance, and this mechanism of affecting absorption is desirable because it may reduce the potential toxicity of the active substance.

The second is based on the fact that larger quantities of substances affect the oral mucosal surface, increasing membrane fluidity, forming transient hydrophilic pores or opening tight junctions. It should be stressed that this effect is not necessarily harmful. The ideal absorption-facilitating compound should include the following properties. It should be pharmacologically inert, non-allergenic, non-toxic and non-irritant, and at the same time highly effective. They should also be compatible with a wide range of medicinal substances and excipients, colourless, tasteless and odourless, as well as cheap and readily available in high purity and acceptable to regulatory authorities worldwide.

Nitrogen azane-type agents with contents ranging from 0.01 to 3 parts by weight may be used as agents to enhance absorption across the mucosal barrier. Such a substance is, for example, ammonium glycyrrhizinate, since glycyrrhizin is the main constituent of liquorice (*Glycyrrhiza glabra*). It is a saponin that is about 50 times sweeter than sucrose. Glycyrrhizinate ammonium is composed of a molecule of glycyrrhizin, glycine and methionine. It has significant antioxidant, anti-inflammatory and antinociceptive effects. Ammonium glycyrrhizinate increases the penetration across cell membranes for lower molecular weight substances without dependence on pH and ionisation state.

An essential component of the invention is the use of a mixture of fillers with different physicochemical properties and substances promoting the absorption of nicotine in the mouth in the form of a complex filler, to which nicotine salt mixtures are added at each step of the method of manufacture.

The preparation of the filler mixture is based on a fiber, a high active surface area fiber, a natural ionex, an organosilicate and substances promoting nicotine absorption in the mouth. All ingredients are in solid and powdered form without the need for further processing.

A form of plant fibre such as cellulose, inulin, arabinogalactan, glucomannan, arabinoxylan or other plant fructooligosaccharides, galactooligosaccharides or xylooligosaccharides can be used as a filler.

Mixing of all the solid powder components produces a filler mixture containing conventional powdered fibre particles with a particle size on the sieve of which 8 weight percent has a particle size on the sieve of less than 60 µm and of which more than 45 weight percent has a particle size on the sieve of more than 400 µm, ranging from 1 to 80 weight parts, and fillers in the form of micropelletised fibre particles ranging from 1 to 75 weight parts, volcanic ash with diatomaceous earth as a substance with high bulk density, colloidal and fluid properties in the range of 0,01 to 10 parts by weight, chitosan as a substance with the character of a natural ion exchange in the range of 0,1 to 5 parts by weight, a mucosal barrier enhancer containing an azane-type nitrogen in the range of 0,01 to 3 parts by weight and a terpene-type mucosal barrier enhancer in the range of 0,01 to 3 parts by weight.

The mixture of nicotine salts contains from 0.1 to 15 parts by weight of electrolyte with a pH of from 2.4 to 12.2 and an oxidation-reduction potential of from minus 900 mV to plus 1200 mV, from 0.1 to 5 parts by weight of nicotine base, from 0.01 to 3 parts by weight of excipients, and from 77 to 99, 79 parts by weight of water.

The method of making the nicotine filler is based on first thoroughly mixing all solid and bulk parts of the filler and excipients. Since some of them are of a powdery nature, have different specific gravity, have different sphericity, i.e. shape differences, or exhibit inter-surface attractive forces, intensive mixing must be carried out by vertical or inclined homogenizers, preferably screw and planetary mixers, which are housed in a hopper with inclined walls. The mixing time depends mainly on the size of the batch, but shall be carried out for at least 30 minutes.

After the mixing of the solid and bulk components of the nicotine formulation has been completed, the liquid mixture of nicotine salts is added under pressure to the mixture thus mixed and is conveyed to the surface of the mixed solids by pumps so that the contents of the inclined wall hopper are as evenly as possible covered with the liquid mixture of nicotine salts. Further intensive mixing is then carried out, with the proviso that the resulting moisture content of the homogeneous nicotine formulation filling must be achieved within a range of up to, as otherwise it would be difficult to optimally feed this filling into the nicotine sachets which are the final product for the consumer of the nicotine product.

### Example number 1

First, a mixture of the solid components of the nicotine product filling is prepared by mixing 8.02 parts by weight of methyl cellulose, 1.5 parts by weight of methyl cellulose in the form of micropellets, 0.3 parts by weight of chitosan ion, 0.03 parts by weight of volcanic ash with diatomaceous earth, followed by the addition of 0.05 parts by weight of azan (monoammonium glycyrrhizinate) and 0.1 parts by weight of Korean pine needle extract with terpenes.

All the bulk solids are homogenised in a hopper with inclined walls by means of a vertical screw and planetary mixer for at least 30 minutes.

The final homogenisation of the solid mixture is checked on a sieve with a minimum diameter of 0,1 mm or more. Subsequently, the solution of the nicotine salt mixture is added by means of a pump through at least three nozzles located in the homogenizer to the entire volume of the mixture with a resulting maximum moisture content of up to 50% for a mixing time of at least 30 min.

Finally, the homogenization of the solid-liquid mixture is checked on a sieve with a diameter of at least 0,1 mm or more.

### Example number 2

Firstly, a filler mixture is prepared by mixing 8.325 parts by weight of methyl cellulose, 1 part by weight of methyl cellulose in the form of micropellets, 0.5 parts by weight of chitosan ionex, 0.025 parts by weight of volcanic ash with diatomaceous earth, and then adding 0.1 parts by weight of azan (*monoammonium glycyrrhizinate*) and 0.05 parts by weight of Korean pine needle extract with terpenes.

All the bulk substances are homogenised by means of a vertical homogeniser. The preparation of the solid mixture of fillers is carried out in the vertical homogenizer for at least 30 min. Subsequently, the solution of the nicotine salt mixture is added by means of a pump through at least three nozzles located in the homogenizer to the entire volume of the mixture with a resulting maximum moisture content of up to 50% for a mixing time of at least 30 min.

Finally, the homogenisation of the solid-liquid mixture is checked on a sieve with a minimum diameter of 0,1 mm or more

### Example number 3

First, a filler mixture is prepared by mixing 5.5 parts by weight of methyl cellulose, 3 parts by weight of methyl cellulose in the form of micropellets, 1 part by weight of chitosan ionex, 0.1 parts by weight of volcanic ash with diatomaceous earth, followed by the addition of 0.15 parts by weight of azan (*monoammonium glycyrrhizinate*) and 0.25 parts by weight of Korean pine needle extract with terpenes.

All the bulk substances are homogenised via a vertical homogeniser for at least 30 minutes.

The final homogenisation is checked on a sieve with a minimum diameter of 0,1 mm or more.

The exemplary embodiment of the invention, which relates to both the mixture of nicotine salts and the method of producing it, including the stability of these mixtures of nicotine salts and their solubility in water, predetermines the physicochemical properties of the critical components of these mixtures, which are crucial to achieve the desired effect.

An essential component of the invention is the use of a reagent or solvent in the form of an anolyte or catholyte solution, to which all the components of the product are added at each step of the method of producing the mixture of nicotine salts.

The preparation of the aqueous solution of catholyte or anolyte is based on water which is usually demineralized using a suitable well-known softener, preferably a catex softener, for example a physical water softener containing a strongly acidic catex resin for the removal of calcium and magnesium salts from the water. The water is then enriched with NaCl. The salt solution enters two chambers separated by a semi-permeable membrane which allows the migration of ions between the chambers. For example, a unidirectional semi-permeable membrane is suitable. One of the chambers contains the cathode, i.e. the electrode with the negative polarity of the source, and the other chamber contains the anode, i.e. the electrode with the positive polarity of the source.

As positive ions flow through the chambers, they are attracted to the negative electrode and receive electrons from the cathode. Neutral atoms or molecules are formed. The positive electrode, i.e. the anode, attracts negatively charged ions, i.e. the anions, and they pass their additional electrons to the anode. Electrolysis gives rise to the ions H+ and OH-, the radicals K and OH, H₂, O₂, HO₂, O₃ and possibly others.

A superoxidized solution is formed, that is, an electrolyte containing the oxidizing agents, hypochlorous acid HOCl and hypochlorite ions OCl-. The desired parameters of the resulting electrolyte are a pH in the range from 2,4 to 12,2 and an oxidation-reduction potential in the range from minus 900 mV to plus 1200 mV. Negative values of the catolyte produce predominantly unstable hydrogen.

The nicotine base is another essential component of the mixture of nicotine salts and the method of its production. Pure nicotine or nicotine base is a colourless liquid with a typical odour. When exposed to air, light or left in a sealed bottle, it changes from a colourless to or pale yellow oily liquid to a brown liquid whose toxicity remains the same. The boiling point of nicotine is 246-247°C. Nicotine is very soluble in polar solvents such as water, methanol, acetonitrile or chloroform. Due to the asymmetric carbon content of the nicotine molecule, it occurs in two enantiomeric forms. The pyrrolidine and pyridine nitrogen content is dibasic at 15°C. At a pH of 7.4 and a temperature of 37°C, about 69% of the pyrrolidine nitrogen is ionized, while the pyridine nitrogen remains non-ionized. Nicotine at pH 7.4 exists in both forms, the non-ionized form penetrates the lipoprotein membrane well, the ionized form does not penetrate the membrane. The degree of ionisation is therefore dependent on the pH. At pH 7,4 the ratio of the ionised to the non-ionised form is 2:1, as with other alkaloids in this group. The pharmacological properties of nicotine are therefore dependent on its stereoisomeric arrangement. The optimum value of actual acidity in terms of maximum solubility and bioavailability of nicotine is at pH 8 to 9.

The excipients are preferably selected from the group consisting of monoterpenes, diterpenes, triterpenes, cyclic terpenes, carboxylic acid esters, cyclic ketones, In most cases, the water is demineralized before use.

An aqueous solution of anolyte or catholyte, i.e. electrolyte and nicotine to form nicotine salts, is used for the production of application units, i.e. most often sachets of the product together with excipients from the group of monoterpenes, diterpenes, triterpenes, cyclic terpenes, carboxylic acid esters, cyclic ketones and filler.

### Example number 4

First, an electrolyte is prepared by mixing 500 ml of a solution from the anode chamber, i.e. an anolyte with pH 2,4 and an oxidation-reduction potential of 1150 mV, and 580 ml of a solution from the cathode chamber, i.e. a catolyte with pH 9,45 and a negative oxidation-reduction potential of 300 to 400 mV. The resulting electrolyte mixture has a pH of 8,5 and an oxidation-reduction potential of minus 857 mV.

The electrolyte thus prepared with the above parameters, i.e. 10 parts by weight, is mixed with 85 parts by weight of water, which is demineralised if necessary, for example with a water softener containing a strongly acidic catex resin to remove calcium and magnesium salts from the water, for example BlueSoft Eco.

Subsequently, excipients are added to the electrolyte-water solution, i.e. in this particular case 1 part by weight of glycerol, 0.1 parts by weight of menthol, 0.02 parts by weight of menthone, 0.005 parts by weight of methyl salicylate, 0.3 parts by weight of xylitol and 0.25 parts by weight of cineol.

The insoluble excipients, such as menthol, are previously converted via a high-speed blender to a suspension by blending a portion of the 85 parts by weight of water together with the crystalline menthol for 5 minutes. Otherwise, the menthol crystals would remain on the surface of the prepared mixture.

Last, 3 parts by weight of nicotine base are added to the electrolyte, which is done without light. The final solution is mechanically stirred for 20 minutes. The solution thus prepared is then left to mature for 20 hours at a stable temperature of up to 20 degrees Celsius.

The prepared mixture of nicotine salts is then filtered to remove impurities and suspended solids exceeding 0,1 millimetre in size.

### Example number 5

First, the electrolyte is prepared by mixing 500 ml of the solution from the anode chamber, i.e. anolyte with pH 2,4 and an oxidation-reduction potential of 1150 mV, and 490 ml of the solution from the cathode chamber, i.e. catolyte with pH 12,2 and a negative oxidation-reduction potential of 400 to 800 mV. The resulting electrolyte mixture exhibits a pH of 9.45 and an oxidation-reduction potential of minus 752 mV.

The electrolyte prepared in this way with the above parameters, i.e. 4 parts by weight, is mixed with 96 parts by weight of water, which is demineralised if necessary, for example with a water softener containing a strongly acidic catex resin to remove calcium and magnesium salts from the water, which may be BlueSoft Eco, for example.

Subsequently, excipients are added to the electrolyte-water solution, in this particular case 1 weight percent glycerol, 0.05 weight percent menthol, 0.02 weight percent menthone, 0.001 weight percent methyl salicylate, 0.2 weight percent xylitol, and 0.3 weight percent cineol.

The insoluble excipients, such as menthol, are previously converted by means of a high-speed mixer into a suspension by mixing a portion of 96 parts by weight of water together with the crystalline menthol for 4 minutes. Otherwise, the menthol crystals would remain on the surface of the prepared mixture.

Lastly, 1,6 parts by weight of nicotine base is added to the electrolyte, which is done without access to light. The final solution is mechanically stirred for 15 minutes. The solution thus prepared is then left to mature for 24 hours at a stable temperature of up to 20 degrees Celsius.

The prepared mixture of nicotine salts is then filtered to remove impurities and suspended solids exceeding 0,1 millimetre in size.

### Example number 6

First, the electrolyte is prepared by mixing 500 ml of the solution from the anode chamber, i.e. anolyte with pH 2,4 and an oxidation-reduction potential of 1150 mV, and 580 ml of the solution from the cathode chamber, i.e. catolyte with pH 9,45 and a negative oxidation-reduction potential of 300 to 400 mV. The resulting electrolyte mixture exhibits a pH of 8.5 and an oxidation-reduction potential of minus 857 mV.

The electrolyte thus prepared with the above parameters, i.e. 12 parts by weight thereof, is mixed with 86 parts by weight of water, which is demineralised if necessary, for example with a water softener containing a strongly acidic catex resin for removing calcium and magnesium salts from water, which may be BlueSoft Eco for example.

Subsequently, excipients are added to the electrolyte-water solution, in this particular case 2 weight percent glycerol, 0.045 weight percent menthol, 0.025 weight percent menthone, 0.02 weight percent methyl salicylate, 0.3 weight percent xylitol, and 0.3 weight percent cineol.

The insoluble excipients, such as menthol, are previously converted via a high-speed blender to a suspension by blending a portion of the 86 parts by weight of water together with the crystalline menthol for 6 minutes. Otherwise, the menthol crystals would remain on the surface of the prepared mixture.

Lastly, 0.56 parts by weight of nicotine base is added to the electrolyte, which is done without access to light. The final solution is mechanically stirred for 25 minutes. The solution thus prepared is then left to mature for 30 hours at a stable temperature of up to 20 degrees Celsius.

The prepared mixture of nicotine salts is then filtered to remove impurities and suspended solids exceeding 0,1 millimetre in size.

### Industrial Applicability

The nicotine filling, in particular the nicotine sachet and the method of manufacture thereof, and the nicotine salt mixture and the method of manufacture thereof according to the present inventions, can be used for the manufacture of final products for consumption by nicotine users in a number of forms, for example in sachet form, but also in powder, tablet, dragee, capsule, lozenge, pellet or microparticle form, where the nicotine salts enter the user's body through the lining of the mouth, taking advantage of the increased stability of nicotine and its absorption.

## Claims

1. A filling of a nicotine formulation, in particular a sachet, **characterized in that** it contains 0.0001 to 10 parts by weight of a mixture of nicotine salts comprising from 0.1 to 15 parts by weight of an electrolyte having a pH of from 2.4 to 12.2 and an oxidation-reduction potential of from minus 900 mV to plus 1200 mV, from 0.1 to 5 parts by weight of nicotine base, from 0, 01 to 3 parts by weight of excipients and 77 to 99.79 parts by weight of water, 1 to 80 parts by weight of filler, 0.1 to 5 parts by weight of surfactants, 0.01 to 10 parts by weight of nicotine formulation fluid and colloidal enhancement agents, and 0.01 to 3 parts by weight of nicotine formulation absorption enhancement agents.

2. The filling of a nicotine formulation, in particular a sachet, according to claim 1, **characterized in that** the filler is a vegetable fibre, in particular cellulose, inulin, arabinogalactan, glucomannan, arabinoxylan or vegetable fructooligosaccharides, galactooligosaccharides or xylooligosaccharides.

3. The filling of a nicotine formulation, in particular a sachet, according to claim 2, **characterized in that** the filler or part thereof is in the form of micropelletized particles having a size of from 1 to 1000 µm.

4. The filling of a nicotine formulation, in particular a sachet, according to claims 1 and 2, **characterized in that** less than 8 weight percent of the filler has a particle size on the sieve of less than 60 micrometers and more than 45 weight percent of the filler has a particle size on the sieve of more than 400 micrometers.

5. The filling of a nicotine formulation, in particular a sachet, according to claim 1, **characterized in that** the active surface substance is chitosan having the character of a natural ion exchange with a degree of deacetylation from 50% to 90%.

6. The filling of a nicotine formulation, in particular the sachet, according to claim 1, **characterized in that** the substance for improving the fluidic and colloidal properties of the nicotine formulation is volcanic dust with a proportion of diatomaceous earth in the range of 50 to 70 weight percent.

7. The filling of a nicotine formulation, in particular a sachet, according to claim 1, **characterized in that** the substance for improving the absorbability of the nicotine mixture is a substance of terpenic character, in particular Korean pine needles and substances containing azane-type nitrogen.

8. A method of manufacturing a filling of a nicotine formulation, in particular a nicotine sachet, **characterized in that** the individual bulk fractions of the nicotine formulation, i.e. the filler, are first mixed with active surface substances, agents to improve the fluid and colloidal properties and agents to improve the absorbability of the nicotine salt mixture by means of at least one vertical or inclined homogenizer, and then spraying the surface of the already homogenized powder fractions with the liquid nicotine salt mixture, comprising from 0,1 to 15 parts by weight of an electrolyte having a pH of from 2,4 to 12,2 and an oxidation-reduction potential of from minus 900 mV to plus 1200 mV, from 0,1 to 5 parts by weight of nicotine base, from 0,01 to 3 parts by weight of excipients, and 77 to 99, 79 parts by weight of water to produce a bulk nicotine formulation having a moisture content of from 1 to 50%.

9. The method of manufacturing a filling of a nicotine formulation, in particular a nicotine sachet, according to claim 8, **characterized in that** the individual bulk fractions of the nicotine formulation are vigorously mixed by an auger agitator with a range of 40 to 200 rpm, wherein the speed of the planetary motion of the mixer arm is from 1 revolution to 10 revolutions per minute, and wherein mixing is carried out throughout the volume for at least 30 minutes by at least one vertical or inclined screw and planetary mixer.

10. The method of manufacturing a filling of a nicotine formulation, in particular a nicotine sachet, according to claim 8, **characterized in that** the intermixed bulk fractions of the nicotine formulation are intensively mixed for at least 30 minutes with a liquid mixture of nicotine salts.

11. A nicotine salt mixture, **characterized in that** it comprises from 0.1 to 15 parts by weight of an electrolyte having a pH of from 2.4 to 12.2 and an oxidation-reduction potential of from minus 900 mV to plus 1200 mV, from 0.1 to 5 parts by weight of nicotine base, from 0.01 to 3 parts by weight of excipients, and 77 to 99, 79 parts by weight of water.

12. The nicotine salt mixture according to claim 11, **characterized in that** the excipients are at least one substance selected from the group consisting of monoterpenes, diterpenes, triterpenes, cyclic terpenes, carboxylic acid esters and cyclic ketones.

13. A method of producing a mixture of nicotine salts, **characterized in that** an electrolyte having a pH of from 2.4 to 12.2 and an oxidation-reduction potential of from minus 900 mV to plus 1200 mV is mixed with water, nicotine base and excipients, wherein the water-insoluble excipients are converted to a suspension and said mixture is then allowed to age at a temperature of up to 20 degrees Celsius for from six to forty-eight hours.

14. The method of producing a mixture of nicotine salts, according to claim 13, **characterized in that** the prepared mixture is stirred vigorously for at least 15 minutes before maturation in its entire volume.

15. The method of producing a mixture of nicotine salts, according to claim 13, **characterized in that** the nicotine base is added to the electrolyte without access to light.

16. The method of producing a mixture of nicotine salts, according to claim 13, **characterized in that** the prepared mixture is filtered after maturation to remove undissolved solids having a grain size greater than 0.1 millimeter.

17. The method of producing a mixture of nicotine salts, according to claim 13, **characterized in that** the water is demineralized with a water softener containing a strong acid catex resin before use in the nicotine salt mixture.
